# EUROPÄISCHE PATENTSCHRIFT

(11) **EP 2 796 101 B1**
(45) Veröffentlichungstag und Bekanntmachung des Hinweises auf die Patenterteilung: **20.04.2016**
(21) Anmeldenummer: 13164960.0
(22) Anmeldetag: 23.04.2013
(51) Int. Cl.: A61B 17/22, A61K 47/36, A61L 24/00, A61L 24/04

(54) **Kit zum Herstellen eines vernetzten Gels zum Umschließen von Nierensteinen und/oder Nierensteinfragmenten**
Kit for producing a cross-linked gel for encapsulating kidney stones and/or kidney stone fragments
Kit de fabrication d'un gel réticulé pour entourer des calcules rénaux et/ou des fragments de calculs rénaux

(43) Veröffentlichungstag der Anmeldung: 29.10.2014
(73) Patentinhaber: Fraunhofer-Gesellschaft zur Förderung der angewandten Forschung e.V., 80686 München (DE); Albert-Ludwigs-Universität Freiburg, 79085 Freiburg (DE)
(72) Erfinder: Grunwald, Ingo, Dr., 28865 Lilienthal (DE); Richter, Katharina, Dr., 28195 Bremen (DE); Miernik, Arkadiusz, Dr., 79104 Freiburg (DE); Schoenthaler, Martin, Dr., 79100 Freiburg (DE)
(74) Vertreter: Eisenführ Speiser

(56) Entgegenhaltungen:
- WO-A2-2009/070766
- US-A1- 2002 119 116
- US-A1- 2012 108 676
- P.-E. LE RENARD ET AL.: "The in vivo performance of magnetic particle-loaded injectible, in situ gelling, carriers for the delivery of local hyperthermia", BIOMATERIALS, Bd. 31, 2010, Seiten 691-705, XP002712366,

## Beschreibung

Die vorliegende Erfindung betrifft primär ein Kit zum Herstellen eines vernetzten Gels enthaltend magnetisierbare Teilchen zum teilweisen oder vollständigen Umschließen von Nierensteinen und/oder Nierensteinfragmenten im Körper, wobei das Kit zudem eine Magnetangel zum Entfernen eines magnetischen Nierenstein-Gel-Verbunds enthält.

Insbesondere betrifft die vorliegende Erfindung ein Kit, umfassend eine Zusammensetzung (A), enthaltend ein oder mehrere kationisch vernetzbare Polymere, und eine Zusammensetzung (B), enthaltend ein oder mehrere Vernetzungsmittel zum Vernetzen des bzw. der kationisch vernetzbaren Polymere, wobei die Zusammensetzung (A) und/oder die Zusammensetzung (B) zudem magnetisierbare Teichen enthält oder das Kit zudem eine Zusammensetzung (C) umfasst, die magnetisierbare Teilchen enthält, und wobei das Kit zudem eine Magnetangel zum Entfernen eines magnetischen Nierenstein-Gel-Verbunds enthält. Bei Kontakt der Zusammensetzung (A) mit der Zusammensetzung (B) in einem Bereich der Niere, der Nierensteine und/oder Nierensteinfragmente enthält, entsteht ein vernetztes Gel, das die Nierensteine und/oder Nierensteinfragmente teilweise oder vollständig umschließt und das die magnetisierbaren Teilchen enthält. Unter Ausnutzung magnetischer Wechselwirkungen kann das Gel mitsamt den Nierensteinen und/oder Nierensteinfragmenten aus dem Körper entfernt werden.

Weitere Aspekte der vorliegenden Erfindung ergeben sich aus der nachfolgenden Beschreibung, insbesondere den Beispielen, sowie den beigefügten Patentansprüchen.

Nierensteine, medizinisch als Nephrolithen bezeichnet, sind Ablagerungen ausgefallener Salze in den Nierengängen oder ableitenden Harnwegen (Harnsteine), die dort häufig starke Schmerzen (Nierenkolik) verursachen. In der überwiegenden Zahl der Fälle handelt es sich um Kalziumoxalatsteine. Daneben kommen aber auch Harnsäuresteine (Urate), Magnesiumammoniumphosphatsteine und Calciumphosphatsteine, sowie weitere mit geringerer Häufigkeit auftretende Zusammensetzungen vor.

Etwa 30 Millionen Menschen in Europa leiden unter Nierensteinen (ca. 5% der Bevölkerung), und die Häufigkeit von Harnsteinerkrankungen in den industrialisierten Ländern zeigt eine ansteigende Tendenz. Besonders hoch (etwa 60%) ist das Risiko, nach Heilung wiederholt von Nierensteinen betroffen zu sein. Medizinische Komplikationen, die im Zusammenhang mit Nierensteinen auftreten können, sind Nierenfunktionsverlust und Infektionskomplikationen bis hin zur Blutvergiftung. Die entstehenden Belastungen für die Gesundheitssysteme sind gravierend.

Kleine Nierensteine wandern häufig von selbst über den Harnleiter in die Harnblase. Erfolgt kein spontaner Abgang, müssen die Steine mit Hilfe anderer Techniken entfernt werden.

Eine Möglichkeit, die Position oder Verteilung von Substanzen oder Objekten im Körper gezielt zu steuern, ist die Ausnutzung magnetischer Wechselwirkungen. Dazu müssen die Zielsubstanzen bzw. Zielobjekte entsprechend magnetisiert werden. Magnetische (Nano-)Partikel haben sich dazu bereits in verschiedenen biomedizinischen Anwendungen als geeignet erwiesen, da sie eine hohe Biokompabilität aufweisen und mit verschiedenen funktionellen Gruppen modifiziert werden können. So werden magnetische Partikel beispielsweise verwendet, um Wirkstoffe im Körper an ihren gewünschten Wirkort zu transportieren. Therapeutische und diagnostische Substanzen können somit effizient eingesetzt werden, und Schäden durch eventuelle Nebenwirkungen in gesunden Geweben werden minimiert. US 2007/0231393 beschreibt in diesem Zusammenhang ein Verfahren, bei dem magnetische Wirkstoffträger-Partikel mittels eines äußeren magnetischen Feldes im Körper positioniert werden.

US 2009/0136594 beschäftigt sich mit einer Methode, biologische Partikel zu magnetisieren, indem sie mit magnetischen Partikeln in Kontakt gebracht werden, die so modifiziert sind, dass sie spezifisch an die biologischen Partikel binden können. Als eine mögliche Anwendung des beschriebenen Verfahrens können Nierensteine oder deren Fragmente magnetisiert werden, um sie mittels einer Vorrichtung, die solche Partikel magnetisch anzieht, aus dem Körper zu entfernen. Um Kalzium-basierte Biomineralien (wie z.B. Nierensteine) spezifisch zu binden, werden die Partikel mit bestimmten Kalzium-bindenden Proteinen oder Proteinfragmenten modifiziert.

Größere Steine können in der Regel nicht durch einen minimal-invasiven Eingriff entfernt werden und müssen daher zunächst in kleinere Bruchstücke zertrümmert bzw. ganz oder zumindest teilweise aufgelöst werden. Eine Methode zur Behandlung von Nierensteinen durch gezieltes Auflösen der Ablagerungen unter Verwendung von quartären Ammoniumsalzen wird beispielsweise in US 5,244,913 beschrieben.

Eine weitere Möglichkeit der Behandlung von Nierensteinen ohne vorhergehendes Zertrümmern wird in US 2006/0269512 angegeben. Hierbei wird die natürliche Peristaltik dazu ausgenutzt, einen Polymerpfropfen durch ein Lumen zu pressen, und dabei den Stein aus dem Lumen zu entfernen. Der Polymerpfropfen kann in situ durch Temperatur- oder pH-Änderung oder durch ionische Wechselwirkungen gebildet werden.

Bei der Lithotripsie werden Nierensteine durch extrakorporale Stoßwellen oder endoskopisch eingeführte Laser- oder Druckluftsonden zertrümmert. Dabei entstehen unterschiedlich große Bruchstücke, die entweder mit Hilfe von Greifwerkzeugen entfernt oder ausgespült werden können. Ein bei der Lithotripsie auftretendes Problem ist, dass sich die Fragmente während des Zertrümmerns verteilen und dabei umgebendes Gewebe schädigen können oder in schwer zugängliche Regionen gelangen.

WO 2005/037062 betrifft eine Methode, mit der Nierensteine mit Hilfe eines Polymerpfropfens in einem bestimmten Bereich eingeschlossen (nicht umschlossen) werden, wodurch Gewebeschäden durch die entstehenden Fragmente während der Zertrümmerung weitgehend verhindert werden können. Gemäß WO 2005/037062 wird auf zumindest einer Seite eines Nierensteins eine gelbildende Flüssigkeit in das Lumen injiziert, beispielsweise ein thermosensitives Polymer, welches bei Körpertemperatur einen Gelpfropfen bildet. Das Polymer kommt dabei in der Regel nicht in Kontakt mit dem Nierenstein, dient aber dazu, die Effizienz der Lithotripsie zu erhöhen, indem es ein Verschieben des Nierensteins verhindert, und das umgebende Gewebe vor Schädigung durch die Fragmentierung schützt.

Gemäß US 2008/0103481 wird ein biokompatibler Polymerpfropfen insbesondere dazu verwendet, um ein Rückwärtsverschieben von Nierensteinen oder Nierensteinfragmenten während der Lithotripsie zu verhindern und dadurch die Schädigung des umgebenden Gewebes zu minimieren.

Ein Ansatz um Objekte, wie beispielsweise Blutgerinnsel, unter Verwendung eines Klebstoffes aus dem Körper zu entfernen, wird in US 2008/0065012 angegeben. Der Klebstoff wird dabei auf einer Oberfläche verteilt und mit Hilfe eines Katheters in den Körper eingeführt. Wenn das Objekt an der Oberfläche angehaftet ist, wird der Katheter wieder herausgezogen und nimmt das Objekt mit.

Auf biologischen Makromolekülen basierende Klebstoffe und insbesondere gelbildende Polymer-Systeme finden in der Medizintechnik zunehmend Anwendungen. Dabei ist ihre hohe Biokompatibilität eines der wichtigsten Auswahlkriterien.

Thermosensitive oder ionisch polymerisierbare Polymere werden beispielsweise verwendet, um den Blutfluss aus verletzen Blutgefäßen zu stoppen. WO 2008/103891 gibt eine Methode an, bei der der Ausfluss von biologischen Flüssigkeiten aus Gewebe oder Gefäßen durch in situ Bildung eines Polymerpfropfens kontrolliert werden kann.

WO 010544 betrifft einen adhäsiven Proteinschaum und dessen Verwendung für chirurgische und therapeutische Anwendungen. Der Schaum besteht aus einer flüssigen Proteinmatrix und einem biokompatiblen Gas und dient dazu, verletztes Gewebe abzudecken bzw. zu schützen oder implantiertes Gewebe mit biologischem Gewebe zu verbinden.

WO 02/18448 beschreibt die pharmazeutische Verwendung von percarboxylierten Polysacchariden bei der Herstellung von Biomaterialien für chirurgische und biomedizinische Anwendungen. Solche Materialien sind besonders geeignet für den Einsatz im Körper, da sie als körpereigen erkannt werden und keine Immunabstoßungsreaktionen hervorrufen. Sie können daher als Beschichtung für Implantate verwendet werden.

Eine Methode zur Verkapselung von Nierengewebe in Kugeln aus biokompatiblen Polymeren wird in US 2009/0162411 beschrieben. Ziel einer solchen Verkapselung ist es, Nierengewebs-Implantate zu erhalten, die einem Patienten, der unter einer Störung der Nierenfunktion leidet, injiziert werden können, um die Funktion der Niere zu unterstützen.

Kalziumalginat als biokompatibles Hyrdogel-Polymer zum Schließen von Schädelöffnungen nach einer Operation am offenen Gehirn wird in WO 2004/080343 offenbart.

Die Eignung von Polysaccharid-haltigen Polymeren für die Bindung biologisch aktiver Moleküle oder ganzer Zellen im Bereich der Organtransplantation und des künstlichen Gewebeersatzes wird in WO 1998/012228 beschrieben.

Alginate werden im medizinischen und kosmetischen Bereich auch als Füllstoffe für die Unterstützung von Haut und Muskeln verwendet. In US 2011/0097367 werden Anwendungen beschrieben, bei denen monolithische Alginat-Implantate durch Injektion einer reinen hochmolekularen Alginat-Lösung ins Gewebe und spontane Vernetzung in situ gebildet werden. Die Vernetzung erfolgt durch Ca²⁺⁻-Ionenbrücken ohne dass zusätzlich Vernetzungsmittel hinzugegeben werden müssen. Die beschriebenen Alginat-Implantate eignen sich für die Behandlung von Falten oder verschiedenen Krankheiten, bei denen die Muskelstruktur geschwächt ist.

In US 6,663,594 B2 wird ein Verfahren zur Immobilisation eines Objekts, beispielweise eines Nierensteins, im Körper beschrieben, bei dem eine gelbildende Flüssigkeit in den Körper injiziert wird. In Kontakt mit dem Objekt wird ein Gel gebildet, welches das Objekt zumindest teilweise erfasst und immobilisiert. Die Immobilisierung dient dazu, das Objekt anschließend fragmentieren zu können ohne eine Verteilung der Bruchstücke zu riskieren bzw. um das Objekt bzw. die Bruchstücke mit einem endoskopischen Werkzeug aus dem Körper zu entfernen. Dabei verhindert das Gel, dass das Objekt bzw. ein Bruchstück verrutscht und von dem Werkzeug nicht erfasst werden kann. Nach Entfernen des Objekts bzw. der Bruchstücke wird das Gel aufgelöst oder mittel eines endoskopischen Werkzeugs extrahiert. Nachteil dieser Methode ist, dass beim Zertrümmern der Nierensteine das bereits abgebundene Gel zerstört werden kann und dadurch wieder Fragmente freigesetzt werden können, oder dass einzelne Fragmente aus dem Polymer austreten. Zudem ist die beschriebene Prozedur sehr aufwendig, da die Steine oder Stein-Fragmente einzeln erfasst und entfernt werden. Im Ergebnis bleiben mit relativ großer Wahrscheinlichkeit einzelne Steinfragmente zurück.

Ein Problem bei der Lithotripsie ist insbesondere das Auftreten mittelgroßer Steinfragmente (insbesondere < 2 mm), auch als "Gries" bezeichnet, da diese Bruchstücke weder effizient gegriffen noch gespült werden können. Restfragmente dieser Größe rutschen durch die Maschen der Greifinstrumente (Fasszängchen oder -körbchen) und machen die Extraktion von Gries sehr zeitaufwändig und bei größeren Steinmassen praktisch undurchführbar. Bisher wurde noch keine Technologie erfolgreich etabliert, um die mittelgroßen Steinfragmente vollständig zu entfernen. Das Zurückbleiben solcher Nierensteinfragmente führt jedoch unweigerlich zur Bildung neuer Nierensteine, da die Bruchstücke bzw. Fragmente als "Kristallisationskeime" fungieren.

Damit ein vollständiges Entfernen von Fragmenten jeglicher Größe sichergestellt werden kann, muss eine einfache Methode entwickelt werden, die dazu geeignet ist, idealerweise alle Bruchstücke zuverlässig zu erfassen. Hierbei sollten vorzugsweise die (zum Teil oben genannten) Probleme und Schwierigkeiten, die im Stand der Technik bekannte Verfahren mit sich bringen, vermieden werden.

Primäre Aufgabe der vorliegenden Erfindung war es, ein Kit bereitzustellen, das dazu geeignet ist, insbesondere Nierensteinfragmente zuverlässig aus dem Körper extrahieren zu können, vorzugsweise mittels minimal-invasiven Verfahrens.

Weitere Aufgaben der vorliegenden Erfindung ergeben sich aus der nachfolgenden Beschreibung sowie insbesondere den beigefügten Patentansprüchen.

Die primäre Aufgabe wird gemäß der vorliegenden Erfindung gelöst durch ein Kit zum Herstellen eines vernetzten Gels zum teilweisen oder vollständigen Umschließen von Nierensteinfragmenten, umfassend
(a) eine Zusammensetzung (A), enthaltend ein oder mehrere kationisch vernetzbare Polymere, und
(b) eine Zusammensetzung (B), enthaltend ein oder mehrere Vernetzungsmittel zum Vernetzen des bzw. der kationisch vernetzbaren Polymere,
   wobei
   die Zusammensetzung (A) und/oder die Zusammensetzung (B) zudem magnetisierbare Teilchen enthält,
   oder
   das Kit zudem eine Zusammensetzung (C) umfasst, die magnetisierbare Teilchen enthält, und wobei das Kit zudem eine Magnetangel zum Entfernen eines magnetischen Nierenstein-Gel-Verbunds enthält.

Im Rahmen der vorliegenden Erfindung sind unter einem "Bereich der Niere" insbesondere die Nierengänge, aber auch andere Bereiche, in denen Nierensteine auftreten können, sowie die ableitenden Harnwege, Harnleiter, Blase oder Harnröhre zu verstehen.

Unter "Nierensteinfragmenten" sind im Zusammenhang mit der vorliegenden Erfindung Bruchstücke von Nierensteinen zu verstehen, die insbesondere durch Zertrümmerung von Nierensteinen (Lithotripsie) entstanden sind.

Durch ein erfindungsgemäßes Einbetten der Nierensteinfragmente und anschließende Extraktion des "Klebstoff-Verbunds" können vorteilhafterweise Fragmente jeglicher Größe vollständig entfernt und damit einer erneuten Steinbildung vorgebeugt werden.

Die Polymere bzw. Polymer-Einheiten der Zusammensetzung (A) werden über ionische Wechselwirkungen (siehe Zusammensetzung (B)) vernetzt. Daher ist eine Vielzahl von Makromolekülen, die als Liganden ein- oder mehrwertiger Kationen auftreten und Chelatkomplexe bilden können, für die erfindungsgemäße Anwendung geeignet. Dazu gehören insbesondere Hydrogele, biokompatible zuckerbasierte (z.B. modifizierte Cellulosen) oder proteinogene Klebstoffe oder fibrin- oder kollagenbasierte Systeme (besonders bevorzugte Polymere sind weiter unten beschrieben).

Beispielsweise polyphenolische Proteine sind in der Lage, über Quervernetzungen ihres Proteingerüsts durch eine Katecholoxidase abzubinden. In vitro können solche Quervernetzungen z.B. auch durch Metallionen erzielt werden. Denkbar ist auch die Verwendung von Hybridsystemen, die auf einer Kombination von synthetischen Polymeren mit phenolischen Aminosäuren beruhen. Die posttranslationale Aminosäure 3,4-Dihydroxyphenylalanin (DOPA) ist beispielsweise auf Grund ihrer vielseitigen Reaktionsmöglichkeiten mit verschiedenen funktionellen Gruppen zur Polymermodifikation besonders geeignet, und entsprechende Gelsysteme zeichnen sich durch verbesserte adhäsive und kohäsive Eigenschaften aus.

Als Vernetzungsmittel der Zusammensetzung (B) dienen vorzugsweise geeignete Kationen. Vorteilhafterweise handelt es sich dabei in der Regel um natürlich in physiologischen Systemen vorkommende Kationen. Es müssen vorteilhafterweise keine zusätzlichen (aggressiven) Reagenzien zugegeben werden, um die Vernetzung unter physiologischen Bedingungen in Gang zu setzen. Zudem entstehen vorteilhafterweise keine unerwünschten Nebenprodukte. Erfindungsgemäß bevorzugt sind solche Systeme, die unter physiologischen Bedingungen abbinden können. Um stabile Vernetzungen über Kationenbrücken auszubilden, ist es vorteilhaft, wenn die Polymere der Zusammensetzung (A) über funktionelle Gruppen (in ausreichender Anzahl) verfügen, die auch bei (leicht) saurem pH als negativ geladene Einheiten vorliegen. In manchen Systemen können beispielsweise der Vernetzungsgrad oder die Geschwindigkeit der Vernetzung über beeinflussbare Größen wie Konzentrationen oder pH-Werte der einzelnen Zusammensetzungen gesteuert werden.

Gemäß einer bevorzugten Ausführung enthält die Zusammensetzung (A) und/oder die Zusammensetzung (B) Chitosan. Bevorzugt enthält die Zusammensetzung (B) Chitosan.

Die Zusammensetzungen (A) und (B) können nacheinander oder gemeinsam eingebracht werden, wobei es bevorzugt ist, dass die Zusammensetzung (B) vor der Zusammensetzung (A) eingebracht wird, um eine geeignete Verteilung und vollständige Einbettung aller Nierensteinfragmente vor Beginn bzw. bei der Vernetzung zu gewährleisten.

Das Abbinden erfolgt bevorzugt in einem Bereich der Niere, in dem Nierensteine und/oder Nierensteinfragmente, insbesondere von mittlerer Größe (vorzugsweise mit einem durchschnittlichen mittleren Durchmesser von 0.1 bis 4 mm, bevorzugt von 0.2 bis 3 mm, besonders bevorzugt von 0.5 bis 2 mm) vorliegen, damit diese vor Ort vollständig oder zumindest teilweise umschlossen werden. Das erfindungsgemäße vernetzte Gel bindet vorzugsweise unter physiologischen Bedingungen ab und weist eine ausreichende Stabilität und Flexibilität auf, um vorzugsweise in einem Stück aus dem Körper extrahiert zu werden.

Ein erfindungsgemäßes System kann zusätzlich weitere Komponenten enthalten. Beispielsweise können den Zusammensetzungen (A) und/oder (B) und/oder einer oder mehreren weiteren Zusammensetzungen eines erfindungsgemäßen Systems Substanzen, die die Gelbildung und/oder die Einbindung der Nierensteinfragmente fördern, zugegeben werden. Solche Substanzen können z.B. Quervernetzer zur Erhöhung der Stabilität des Gels sein.

Gemäß einer bevorzugten Ausführung der vorliegenden Erfindung ist bzw. sind das bzw. ein, mehrere oder sämtliche kationisch vernetzbare Polymere der Zusammensetzung (A) ausgewählt aus der Gruppe bestehend aus Polysacchariden, insbesondere Polysacchariden mit deprotonierten oder deprotonierbaren funktionellen Gruppen, vorzugsweise Carboxy-Gruppen, bevorzugt Polysacchariden aus der Gruppe der Polyuronide, besonders bevorzugt Polysacchariden aus der Gruppe der Alginate und Pektine.

Polysaccharide wie Alginate und Pektine sind für den Einsatz im Körper besonders geeignet, da sie keine inflammatorischen Reaktionen oder Immunabstoßung hervorrufen und ein geringes Risiko eines Gewebetraumas mit sich bringen. Zudem sind sie biologisch abbaubar und verfügen über zahlreiche Carbonsäuregruppen, die Chelatkomplexe mit mehrwertigen Kationen bilden können. Sie sind vorteilhafterweise dazu in der Lage, unter Wasser und bei physiologischen Temperaturen zu vernetzen und können leicht in Lösung gehandhabt werden. Die Vernetzung erfolgt zügig aber ohne dabei feine Nierenkanälchen oder die Endoskopieinstrumente zu verkleben. Die entstehenden Gele weisen eine ausreichende Stabilität und Flexibilität auf, um zusammen mit den Nierensteinfragmenten extrahiert werden zu können.

Gemäß einer weiteren bevorzugten Ausgestaltung der vorliegenden Erfindung ist bzw. sind das bzw. ein, mehrere oder sämtliche Vernetzungsmittel der Zusammensetzung (B) ausgewählt aus der Gruppe bestehend aus zwei- und dreiwertigen Kationen, vorzugsweise Eisen- und Kalzium-Ionen.

Eisen- und Kalziumionen sind in physiologischen Systemen natürlich vorkommende Kationen, die in Form von biologisch verträglichen Lösungen einfach verabreicht werden können. Sie haben eine geeignete Koordinationschemie und können stabile Chelatkomplexe zur Vernetzung ausbilden.

Gemäß einer weiteren bevorzugten Ausgestaltung der vorliegenden Erfindung weist die Zusammensetzung (B) einen sauren pH-Wert auf, vorzugsweise einen pH im Bereich von 3,5 bis 4,5.

Bei einem pH im Bereich von 3,5 bis 4,5 liegen die Kationen der Zusammensetzung (B) frei in Lösung vor und stehen somit zur Komplexierung zur Verfügung. Vorteilhafterweise sind in diesem pH-Bereich aber die am Polysaccharid befindlichen Säuregruppen zu einem großen Teil deprotoniert, wodurch es zu einer effektiven Vernetzungsreaktion kommt. Wird im Bereich der zu entfernenden Nierensteinfragmente eine gepufferte Lösung (bei einem pH von ca. 4) vorgelegt, kommt es infolge des Einleitens der polysaccharidhaltigen Zusammensetzung (A) zu einer Herabsetzung der Löslichkeit (Koazervation). Der Prozess der Koazervation beansprucht eine gewisse Zeit, während der die Nierensteinfragmente eingebettet werden. Vorteilhafterweise ist damit auch die Geschwindigkeit der Vernetzungsreaktion über den pH der verwendeten Zusammensetzungen steuerbar.

Gemäß einer weiteren bevorzugten Ausführung der vorliegenden Erfindung sind die magnetisierbaren Teilchen ausgewählt aus Teilchen enthaltend oder bestehend aus ferromagnetische(n) Elemente(n) wie Eisen, Nickel und Kobalt sowie Legierungen wie AlNiCo, SmCo, Nd₂Fe₁₄B, Ni80Fe20, NiFeCo und/oder deren Oxide wie Eisenoxidpartikel, insbesondere Eisenoxidnanopartikel aus Fe₃O₄ und/oder γ-Fe₂O₃.

Der Zusatz magnetisierbarer Teilchen eröffnet eine vorteilhafte Methode, den abgebundenen "Klebstoff-Verbund" durch Ausnutzung der magnetischen Eigenschaften aus dem Körper zu entfernen.

Eisenoxidpartikel haben sich in medizintechnischen und pharmazeutischen Anwendungen, z.B. als intravenös verabreichtes Kontrastmittel für die Magnetresonanztomographie oder zur Tumortherapie, als geeignet erwiesen. Zur Erhöhung der Biokompatibilität und kolloidalen Stabilität sind solche Partikel meist umhüllt mit z.B. Dextranen, Polyvinylalkoholen, Dimercaptobernsteinsäure u.a..

Die Eisenoxidpartikel verleihen dem Klebstoff außerdem eine dunkle Färbung, was im Gegensatz zu dem unmodifizierten Gel, das nahezu farblos ist, eine einfachere Handhabung nach visuellen Aspekten ermöglicht.

Gemäß einer bevorzugten Ausführung der vorliegenden Erfindung beträgt die Gesamtmenge der magnetisierbaren Teilchen, bezogen auf das Gesamtgewicht der die magnetisierbaren Teilchen enthaltenden Zusammensetzung (A) bzw. (B) bzw. (C), wenigstens 0.1 Gew.-% und liegt vorzugsweise im Bereich von 0.1 bis 70 Gew.-%, besonders bevorzugt im Bereich von 1 bis 11 Gew.-%.

Um zu gewährleisten, dass das Gel umfassend die Nierensteine und/oder Nierensteinfragmente effizient durch Ausnutzung magnetischer Wechselwirkungen aus dem Körper entfernt werden kann, ist es notwendig, eine geeignete Menge an magnetisierbaren Teilchen im vernetzen Gel einzusetzen.

Gemäß einer bevorzugten Ausführung der vorliegenden Erfindung beträgt die Konzentration der in der Zusammensetzung (B) enthaltenen Vernetzungsmittel, bezogen auf das Gesamtvolumen der Zusammensetzung (B), wenigstens 0.1 mol/L und liegt vorzugsweise im Bereich von 0.1 bis 3 mol/L, besonders bevorzugt im Bereich von 0.1 bis 1 mol/L.

Die Menge der vorhandenen Vernetzungsmittel beeinflusst sowohl die Geschwindigkeit der Vernetzungsreaktion, als auch auf die Stabilität und Flexibilität des vernetzten Gels. Die in der oben beschriebenen, bevorzugten Ausführung der vorliegenden Erfindung angegebenen Mengen gewährleisten, dass möglichst schnell ein stabiles und flexibles Gel gebildet wird, welches gegebenenfalls in einem Stück aus dem Körper entfernt werden kann.

In einer weiteren bevorzugten Ausführung liegt die Gesamtmenge an in der Zusammensetzung (A) und/oder der Zusammensetzung (B) vorhandenem Chitosan zwischen 0.05 Gew.-% und 1 Gew.-%, jeweils bezogen auf das Gesamtgewicht der Zusammensetzung (A) bzw. (B).

Gemäß einer weiteren bevorzugten Ausführung der vorliegenden Erfindung enthält die Zusammensetzung (A), die Zusammensetzung (B), gegebenenfalls die Zusammensetzung (C) oder eine weitere, gegebenenfalls in dem Kit enthaltene Zusammensetzung (D) eine oder mehrere Substanzen zum Verbessern der Vernetzung des bzw. der kationisch vernetzbaren Polymere, insbesondere Quervernetzer. Dem Fachmann sind Verbindungen mit funktionellen Gruppen bekannt, aus denen er solche auswählen kann, die geeignet sind die Vernetzung zu verbessern. Beispielsweise können Aminosäuren verwendet werden.

Eine Zugabe von Substanzen, die die Vernetzung des Gels verbessern, sorgt dafür, dass die Nierensteine und/oder Nierensteinfragmente sicher umschlossen werden und das Gel während dem Vorgang der Extraktion nicht reißt. Dadurch wird eine vollständige Entfernung aller Nierensteine und/oder Nierensteinfragmente in einem Extraktionsschritt ermöglicht.

Gemäß einer weiteren bevorzugten Ausführung der vorliegenden Erfindung enthält die Zusammensetzung (A), die Zusammensetzung (B), gegebenenfalls die Zusammensetzung (C) oder eine weitere, gegebenenfalls in dem Kit enthaltene Zusammensetzung (D) oder (E) eine oder mehrere Substanzen zum Verbessern oder Ermöglichen der Ausbildung kovalenter Bindungen zwischen dem vernetzten Gel und den zu umschließenden Nierensteinen und/oder Nierensteinfragmenten.

Substanzen, die die Ausbildung kovalenter Bindungen zwischen dem vernetzten Gel und den zu umschließenden Nierensteinen und/oder Nierensteinfragmenten verbessern oder ermöglichen, gewährleisten, dass auch Nierensteine und/oder Nierensteinfragmente sicher erfasst und mit extrahiert werden, die unter Umständen nicht vollständig umschlossen wurden oder lediglich mit dem vernetzenden Gel in Kontakt gekommen sind. Eine vollständige Entfernung aller Nierensteine und/oder Nierensteinfragmente wird somit sichergestellt.

Gemäß einer weiteren bevorzugten Ausführung der vorliegenden Erfindung enthält die Zusammensetzung (A), die Zusammensetzung (B), gegebenenfalls die Zusammensetzung (C) oder eine weitere, gegebenenfalls in dem Kit enthaltene Zusammensetzung (D), (E) oder (F) eine oder mehrere weitere Substanzen der folgenden Liste: Farbstoffe, Kontrastmittel, Konservierungsmittel und Stabilisatoren.

Der Zusatz von Farbstoffen und/oder Kontrastmitteln zu dem vernetzenden Gel-system kann die Handhabung des vernetzten Gels während dem Vorgang der Extraktion erheblich erleichtern. Das Gel kann dadurch im Körper zu jedem Zeitpunkt der Extraktion lokalisiert werden und die Vollständigkeit der Extraktion kann effizient überprüft werden.

Gemäß der vorliegenden Erfindung enthält das Kit zudem eine Magnetangel zum Entfernen eines magnetischen Nierenstein-Gel-Verbunds.

Für die Magnetangel können beliebige magnetisierbare Materialien verwendet werden. Diese können für die Anwendung mit einer geeigneten Schicht, z.B. aus einem Polymer, ummantelt sein. Mögliche Formen einer Magnetangel umfassen z.B. einen Draht, der in Haken oder Schlaufen geformt sein kann, sowie Netze, Körbchen oder Zangen-förmige Anordnungen.

Eine Magnetangel, die geeignet ist auf endoskopischem Weg in den Körper eingeführt zu werden, ermöglicht eine schonende minimal-invasive Extraktion des Gels. Durch direkten Kontakt zwischen Gel und Angel, kommt es zu magnetischen Wechselwirkungen, die stark genug sind eine sichere Extraktion zu gewährleisten.

Im Folgenden wird die vorliegende Erfindung anhand ausgewählter Beispiele näher erläutert.

### Beispiel 1: Inhalt eines erfindungsgemäßen Kits:

### Zusammensetzung (A): 2 g Alginat in 200 mL Wasser

Zusammensetzung (B): 3 mL einer wasserbasierten Chitosanlösung (0.32 Gew.-%, pH 6), mit ca. 5 Tropfen einer Oxalsäurelösung in Wasser (1 M) sowie 0.5 mL einer wässrigen FeCl₃-Lösung (1 M)

Zusammensetzung (C): 4 bis 40 mM Eisen (0.35 bis 3.5 g pro Liter) enthaltende Partikel-Suspension in Wasser oder in physiologischem Puffer (M. Geppert et al., Nanotechnology 22 (2011) 145101).

Magnetangel, bestehend aus einer PTFE-ummantelten Vanadium-Legierung.

### Beispiel 2: Anwendung eines Kits (Vergleichsbeispiel).

Ein Katheter wird durch den Harntrakt in einen Bereich der Niere, der zu entfernende Nierensteinfragmente enthält, eingebracht. 50 mL einer Zusammensetzung (B) gemäß Beispiel 1 werden durch den Katheter in den Bereich der Niere eingeleitet. Im Anschluss wird eine Zusammensetzung (A) gemäß Beispiel 1 durch den oder einen weiteren Katheter in den Bereich der Niere eingeleitet, wodurch es über einen Zeitraum von ca. 1 min zur Gelbildung kommt. Dann wird ein endoskopisches Greifinstrument über den Harntrakt eingeführt. Mit dem Greifinstrument wird das gefestigte Gel gefasst und durch Extraktion über den Harntrakt aus dem Körper entfernt.

## Patentansprüche

1. Kit zum Herstellen eines vernetzten Gels zum teilweisen oder vollständigen Umschließen von Nierensteinfragmenten, wobei das Kit umfasst,
(a) eine Zusammensetzung (A), enthaltend ein oder mehrere kationisch vernetzbare(s) Polymer(e),
und
(b) eine Zusammensetzung (B), enthaltend ein oder mehrere Vernetzungsmittel zum Vernetzen von einem bzw. mehreren kationisch vernetzbaren Polymer(en),
wobei
die Zusammensetzung (A) und/oder die Zusammensetzung (B) zudem magnetisierbare Teilchen enthält oder wobei das Kit zudem eine Zusammensetzung (C) umfasst, die magnetisierbare Teilchen enthält, und
wobei
(c) das Kit zudem eine Magnetangel zum Entfernen eines magnetischen Nierenstein-Gel-Verbunds enthält.

2. Kit nach Anspruch 1, wobei das eine bzw. die mehreren oder sämtliche kationisch vernetzbare(n) Polymer(e) der Zusammensetzung (A) ausgewählt ist bzw. sind aus der Gruppe bestehend aus Polysacchariden, insbesondere Polysacchariden mit deprotonierten oder deprotonierbaren funktionellen Gruppen, vorzugsweise Carboxy-Gruppen, bevorzugt Polysacchariden aus der Gruppe der Polyuronide, besonders bevorzugt Polysacchariden aus der Gruppe der Alginate und Pektine.

3. Kit nach einem der Ansprüche 1 oder 2, wobei das eine bzw. die mehreren oder sämtliche Vernetzungsmittel der Zusammensetzung (B) ausgewählt ist bzw. sind aus der Gruppe bestehend aus zwei- und dreiwertigen Kationen, vorzugsweise Eisen- und Kalzium-Ionen.

4. Kit nach einem der vorangehenden Ansprüche, wobei die Zusammensetzung (B) einen sauren pH-Wert aufweist, vorzugsweise einen pH im Bereich von 3,5 bis 4,5.

5. Kit nach einem der vorangehenden Ansprüche, wobei die magnetisierbaren Teilchen ausgewählt sind aus Teilchen enthaltend oder bestehend aus ferromagnetische(n) Elemente(n) wie Eisen, Nickel und Kobalt sowie Legierungen wie AlNiCo, SmCo, Nd₂Fe₁₄B, Ni80Fe20, NiFeCo und/oder deren Oxide wie Eisenoxidpartikel, insbesondere Eisenoxidnanopartikel aus Fe₃O₄ und/oder γ-Fe₂O₃.

6. Kit nach einem der vorangehenden Ansprüche, wobei die Gesamtmenge der magnetisierbaren Teilchen, bezogen auf das Gesamtgewicht der die magnetisierbaren Teilchen enthaltenden Zusammensetzung (A) bzw. (B) bzw. (C), wenigstens 0.1 Gew.-% beträgt und vorzugsweise im Bereich von 0.1 bis 70 Gew.-%, besonders bevorzugt im Bereich von 1 bis 11 Gew.-% liegt.

7. Kit nach einem der vorangehenden Ansprüche, wobei die Konzentration der in der Zusammensetzung (B) enthaltenen Vernetzungsmittel, bezogen auf das Gesamtvolumen der Zusammensetzung (B), wenigstens 0.1 mol/L beträgt und vorzugsweise im Bereich von 0.1 bis 3 mol/L, besonders bevorzugt im Bereich von 0.1 bis 1 mol/L liegt.

8. Kit nach einem der vorangehenden Ansprüche, wobei die Zusammensetzung (A), die Zusammensetzung (B), gegebenenfalls die Zusammensetzung (C) oder eine weitere, gegebenenfalls in dem Kit enthaltene Zusammensetzung (D) eine oder mehrere Substanzen zum Verbessern der Vernetzung des bzw. der kationisch vernetzbaren Polymere, insbesondere Quervernetzer, vorzugsweise Aminosäuren, enthält.

9. Kit nach einem der vorangehenden Ansprüche, wobei die Zusammensetzung (A), die Zusammensetzung (B), gegebenenfalls die Zusammensetzung (C) oder eine weitere, gegebenenfalls in dem Kit enthaltene Zusammensetzung (D) oder (E) eine oder mehrere Substanzen zum Verbessern oder Ermöglichen der Ausbildung kovalenter Bindungen zwischen dem vernetzten Gel und den zu umschließenden Nierensteinfragmenten enthält.

10. Kit nach einem der vorangehenden Ansprüche, wobei die Zusammensetzung (A), die Zusammensetzung (B), gegebenenfalls die Zusammensetzung (C) oder eine weitere, gegebenenfalls in dem Kit enthaltene Zusammensetzung (D), (E) oder (F) eine oder mehrere weitere Substanzen der folgenden Liste enthält: Farbstoffe, Kontrastmittel, Konservierungsmittel und Stabilisatoren.

## Claims

1. Kit for producing a crosslinked gel for partly or fully surrounding kidney stone fragments, the kit comprising,
(a) a composition (A), comprising one or several cationically crosslinkable polymer(s),
and
(b) a composition (B), comprising one or several crosslinking agent(s) for crosslinking one or several cationically crosslinkable polymer(s), respectively,
wherein
composition (A) and/or composition (B) additionally comprise(s) magnetizable particles or wherein the kit additionally comprises a composition (C) that comprises magnetizable particles, and
wherein
(c) the kit additionally comprises a magnet fishing instrument for removal of a magnetic kidney stone-gel-composite.

2. Kit according to claim 1, wherein the one or several or all of the cationically crosslinkable polymer(s), respectively, of compound (A) is or are selected from the group consisting of polysaccharides, more particularly polysaccharides with deprotonated or deprotonatable functional groups, preferably carboxy groups, preferably polysaccharides from the group of polyuronides, particularly preferred polysaccharides from the group of alginates and pectins.

3. Kit according to claim 1 or 2, wherein the one or several or all of the crosslinking agent(s), respectively, of compound (B) is or are selected from the group consisting of divalent and trivalent cations, preferably iron and calcium ions.

4. Kit according to any of the preceding claims, wherein composition (B) has an acidic pH-value, preferably a pH in the range of 3.5 to 4.5.

5. Kit according to any of the preceding claims, wherein the magnetizable particles are selected from particles comprising or consisting of ferromagnetic elements such as iron, nickel and cobalt as well as alloys such as AlNiCo, SmCo, Nd₂Fe₁₄B, Ni₈₀Fe₂₀, NiFeCo and/or oxides thereof such as iron oxide particles, more particularly iron oxide nanoparticles made of Fe₃O₄ and/or γ-Fe₂O₃.

6. Kit according to any of the preceding claims, wherein the total amount of the magnetizable particles with regard to the total weight of composition (A) or (B) or (C), respectively, that contain(s) the magnetizable particles, is at least 0.1 wt.-% and is preferably in the range of from 0.1 to 70 wt.-%, particularly preferably in the range of from 1 to 11 wt.-%.

7. Kit according to any of the preceding claims, wherein the concentration of the crosslinking agents that are comprised in composition (B) is, with regard to the total volume of composition (B), at least 0.1 mol/L and preferably is in the range of from 0.1 to 3 mol/L, particularly preferably in the range of from 0.1 to 1 mol/L.

8. Kit according to any of the preceding claims, wherein composition (A), composition (B), composition (C) if applicable or a further composition (D) comprised in the kit if applicable, comprises one or several substances for improving the crosslinking of the cationically crosslinkable polymer(s), more particularly crosslinkers, preferably amino acids.

9. Kit according to any of the preceding claims, wherein composition (A), composition (B), composition (C) if applicable or a further composition (D) or (E) comprised in the kit if applicable, comprises one or several substances for improving or enabling the formation of covalent bonds between the crosslinked gel and the kidney stone fragments that are to be surrounded.

10. Kit according to any of the preceding claims, wherein composition (A), composition (B), composition (C) if applicable or a further composition (D), (E) or (F) comprised in the kit if applicable, comprises one or several further substances from the following list: dyes, contrast agents, preservatives and stabilizers.

## Revendications

1. Kit de préparation d'un gel réticulé destiné à envelopper en partie ou complètement des fragments de calcul rénal, ledit kit comprenant:
(a) une composition (A) contenant un ou plusieurs polymère(s) réticulable(s) de façon cationique,
et
(b) une composition (B) contenant un ou plusieurs agent(s) de réticulation pour réticuler un ou bien plusieurs polymère(s) réticulable(s) de façon cationique,
dans lequel
la composition (A) et/ou la composition (B) contient en outre des particules magnétisables ou dans lequel le kit contient en outre une composition (C) qui contient des particules magnétisables, et
dans lequel
(c) ledit kit comprend en outre un dispositif magnétique à pêcher pour enlever un assemblage magnétique de calcul rénal et de gel.

2. Kit selon la revendication 1, dans lequel ledit un ou bien lesdits plusieurs ou tous les polymère(s) réticulable(s) de façon cationique de la composition (A) est ou bien sont choisi(s) dans le groupe constitué par les polysaccharides, en particulier les polysaccharides comprenant des groupes fonctionnels déprotonés ou aptes à être déprotonés, de préférence des groupes carboxyles, de préférence les polysaccharides provenant du groupe des polyuronides, de manière particulièrement préférée les polysaccharides provenant du groupe des alginates et des pectines.

3. Kit selon l'une quelconque des revendications 1 ou 2, dans lequel ledit un ou bien lesdits plusieurs ou tous les agent(s) de réticulation de la composition (B) est ou bien sont choisi(s) dans le groupe constitué par des cations bi- et trivalents, de préférence des ions de fer et de calcium.

4. Kit selon l'une quelconque des revendications précédentes, dans lequel la composition (B) présente une valeur pH acide, de préférence une valeur pH comprise entre 3,5 et 4,5.

5. Kit selon l'une quelconque des revendications précédentes, dans lequel les particules magnétisables sont choisies parmi des particules comprenant ou constituées par des éléments ferromagnétiques tels que le fer, le nickel et le cobalt ainsi que des alliages tels que AlNiCo, SmCo, Nd₂Fe₁₄B, Ni80Fe20, NiFeCo et/ou des oxydes de ceux-ci, tels que les particules d'oxyde de fer, en particulier les nanoparticules d'oxyde de fer en Fe₃O₄ et/ou γ-Fe₂O₃.

6. Kit selon l'une quelconque des revendications précédentes, dans lequel la quantité totale des particules magnétisables, par rapport au poids total de la composition (A) ou bien (B) ou bien (C) contenant les particules magnétisables, est de 0,1 % en poids au moins et est, de préférence, comprise entre 0,1 et 70 % en poids, de manière particulièrement préférée entre 1 et 11 % en poids.

7. Kit selon l'une quelconque des revendications précédentes, dans lequel la concentration des agents de réticulation contenus dans la composition (B), par rapport au volume total de la composition (B), est de 0,1 mol/L au moins et est, de préférence, comprise entre 0,1 et 3 mol/L, de manière particulièrement préférée entre 0,1 et 1 mol/L.

8. Kit selon l'une quelconque des revendications précédentes, dans lequel la composition (A), la composition (B), le cas échéant la composition (C) ou une autre composition (D) qui est contenue le cas échéant dans ledit kit comprend une ou plusieurs substances destinées à améliorer la réticulation du ou bien des polymères réticulables de façon cationique, en particulier des agents de réticulation transversale, de préférence des acides aminés.

9. Kit selon l'une quelconque des revendications précédentes, dans lequel la composition (A), la composition (B), le cas échéant la composition (C) ou une autre composition (D) ou (E) qui est contenue le cas échéant dans ledit kit comprend une ou plusieurs substances destinées à améliorer ou à permettre la formation de liaisons covalentes entre le gel réticulé et les fragments de calcul rénal à envelopper.

10. Kit selon l'une quelconque des revendications précédentes, dans lequel la composition (A), la composition (B), le cas échéant la composition (C) ou une autre composition (D), (E) ou (F) qui est contenue le cas échéant dans ledit kit comprend une ou plusieurs autres substances de la liste suivante: colorants, agents de contraste, conservateurs et agents stabilisants.
